(19) Europäisches Patentamt
European Patent Office
Office européen des brevets

(11) EP 2 363 387 B1

(12) **EUROPÄISCHE PATENTSCHRIFT**

(45) Veröffentlichungstag und Bekanntmachung des Hinweises auf die Patenterteilung:
**14.12.2016 Patentblatt 2016/50**

(21) Anmeldenummer: **11153483.0**

(22) Anmeldetag: **07.02.2011**

(51) Int Cl.:
***C07C 69/704*** *(2006.01)*
***A61K 8/37*** *(2006.01)*
***A61K 8/39*** *(2006.01)*
***A61Q 5/00*** *(2006.01)*
***A61Q 15/00*** *(2006.01)*
***A61Q 19/00*** *(2006.01)*
***A61K 8/365*** *(2006.01)*
***A61K 8/368*** *(2006.01)*
***C08G 63/668*** *(2006.01)*
***A61Q 17/04*** *(2006.01)*
***A61Q 19/04*** *(2006.01)*

(54) **Partialester eines Polyglycerins mit mindestens einer Carbonsäure und einer polyfunktionalen Carbonsäure, ihre Herstellung und Verwendung**

Partial ester of a polyglycerine with at least once carboxylic acid and a polyfunctional carboxylic acid, production and use of same

Ester partiel d'une polyglycérine dotée d'au moins un acide de carbone et d'un acide de carbone polyfonctionnel, sa fabrication et son utilisation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.03.2010 DE 102010002609**

(43) Veröffentlichungstag der Anmeldung:
**07.09.2011 Patentblatt 2011/36**

(73) Patentinhaber: **Evonik Degussa GmbH**
**45128 Essen (DE)**

(72) Erfinder:
- **Wenk, Hans Henning**
  **45470, Mülheim an der Ruhr (DE)**
- **Meyer, Juergen**
  **45134, Essen (DE)**
- **Bergfried, Stefan**
  **45134, Essen (DE)**
- **Foetsch, Hannelore**
  **45355, Essen (DE)**

(56) Entgegenhaltungen:
**EP-A2- 0 451 461**
**EP-B1- 0 835 862**
**WO-A1-01/72683**
**DE-C1- 4 409 569**

Bemerkungen:
Die Akte enthält technische Angaben, die nach dem Eingang der Anmeldung eingereicht wurden und die nicht in dieser Patentschrift enthalten sind.

EP 2 363 387 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Gebiet der Erfindung

Gegenstand der Erfindung sind spezielle (Poly-)glycerinpartialester mit mindestens einer Carbonsäure und einer polyfunktionalen Carbonsäure sowie deren Verwendung als Emulgator und Formulierungen enthaltend erfindungsgemäße Partialester.

Stand der Technik

In den letzten Jahren ist auf dem Kosmetikmarkt ein starker Trend in Richtung möglichst natürlicher Produkte zu verfolgen. Um diesen erfüllen zu können, ist es notwendig, leistungsstarke Emulgatoren auf Basis nachwachsender Rohstoffe anbieten zu können. Gängige Emulgatoren in der Kosmetik enthalten als hydrophile Gruppen oft Polyethylenglykolgruppen (PEG), die durch Polymerisation von petrochemisch gewonnenem Ethylenoxid hergestellt werden können. Da in möglichst natürlichen Formulierungen alle eingesetzten Rohstoffe auf nachwachsenden Quellen stammen sollten, sind PEG-haltige Emulgatoren in solchen Formulierungen nicht erwünscht.
Polyglycerinester sind ein bevorzugte PEG-freie Alternative für kosmetische Emulgatoren auf Basis nachwachsender Rohstoffe.
Der Einsatz von Polyglycerinestern in Kosmetika als Emulgator ist eine altbekannte Technik.
In EP-B-0 835 862 werden Polyglycerinpartialester beschrieben, die erhältlich sind durch Veresterung eines Polyglyceringemisches mit einem Veresterungsgrad des Polyglycerins zwischen 30 und 75 % und gesättigten oder ungesättigten, linearen oder verzweigten Fettsäuren mit 12 bis 22 C-Atomen und Dimerfettsäuren mit einer mittleren Funktionalität von 2 bis 2,4. Diese besitzen den Vorteil, dass insbesondere die Gefrierstabiltität dieser Emulsionen sehr gut ist. Die Emulsionen sind allerdings noch relativ dickflüssig und sind W/O-Emulsionen, weswegen diese Polyglycerinpartialester sich hauptsächlich zur Herstellung reichhaltiger Lotionen und Cremes eignen.
Eine weitere Alternative für PEG-freie natürliche Emulgatoren sind auch Zitronensäureester.

Der Einsatz von Estern der Zitronensäure in Kosmetika als Emulgator oder Solubilisator ist ebenfalls lange bekannt; so ist etwa der O/W Emulgator Glycerylstearatcitrat, (2-Hydroxy-1,2,3-propantricarbonsäure-1,2,3-propantriolmonooctadecanoat, INCI Glyceryl Stearate Citrate, CAS 39175-72-9), der Citronensäureester des Glycerylstearats, u.a. unter der Bezeichnung AXOL C 62 bei der Firma Evonik Goldschmidt im Handel erhältlich.
Die WO2006034992 und WO2008092676 beschreiben beispielsweise kosmetische O/W-Emulsion enthaltend Glycerylstearatcitrat in Verbindung mit weiteren Emulgatoren.
Die WO2004112731 beschreibt O/W-Emulgator enthaltend Glyceryloleatcitrat und Viskositätsmodifizierer.

Nachteilig bei der Verwendung der Zitronensäureester ist ihre starke Hydrolyseempfindlichkeit, die den Verwendungsspielraum dieser Emulgatoren gewöhnlich auf einen pH-Bereich von 5.5 bis 8 einschränkt. Dies ist insbesondere von Nachteil für die Entwicklung möglichst natürlicher kosmetischer Formulierungen, die beispielsweise Ecocert entsprechen möchten. Für Kosmetikprodukte, die Ecocert-Anforderungen entsprechen sollen, sind für eine Konservierung im Allgemeinen nur organischen Säuren wie Benzoesäure oder Sorbinsäure einsetzbar, die wiederum einen pH-Wert der Emulsionen von 4 - 5 erfordern. Somit können übliche Zitronensäureester in solchen Formulierungen nicht eingesetzt werden.

Einfache Polyglycerinester, wie beispielsweise Polygylcerin-3 Distearate zeichnen sich in der Regel durch eine eingeschränkte Formulierungsflexiblität aus, die sich beispielsweise in Emulsionsinstabilitäten in kritischen Emulsionssystemen zeigt.
Mischester aus Polyglycerin und Methylglucose und Stearinsäure, wie beispielsweise Polyglyceryl-3 Methylglucose Distearate weisen ein hervorragendes Stabilisierungspotential und ein breites Verwendungsspektrum auf. Jedoch sind die in diesen Produkten enthaltenen Methylgruppen auf dem Rohstoff Methanol basiert und sind damit teilweise auch petrochemischen Ursprungs.

Prinzipiell können auch Sorbitan- oder Saccharoseester als O/W Emulgatoren eingesetzt werden. Eine gängige Kombination ist beispielsweise Sorbitan Stearate & Sucrose Cocoate. Allerdings zeichnen sich auch diese Kombinationen in der Regel durch eine eingeschränkte Emulsionsstabilisierung und geringe Formulierungsflexibilität aus.

Die DE4409569 offenbart Polyglycerinpolyricinoleate, dadurch erhältlich, dass man Polyricinolsäure mit einem Eigenkondensationsgrad im Bereich von 2 bis 10 mit einem Polyglyceringemisch der Zusammensetzung

Glycerin : 5 bis 35 Gew.-%
Diglycerine : 15 bis 40 Gew.-%
Triglycerine : 10 bis 30 Gew.-%
Tetraglycerine : 8 bis 20 Gew.-%
Pentaglycerine : 3 bis 10 Gew.-%

Oligoglycerine : ad 100 Gew.-%
in an sich bekannter Weise verestert.

Die EP451461 offenbart die Verwendung von Mischungen aus Polyglycerinfettsäureestern, erhältlich durch partielle Veresterung von bestimmten Polyglycerinmischungen mit mindestens einer gesättigten Fettsäure mit 12 bis 22 C-Atomen oder mindestens einer ungesättigten Fettsäure mit 16 bis 22 C-Atomen, wobei die eingesetzte ungesättigte Fettsäure oder Fettsäuremischung noch bis zu 10 Gew.-% an gesättigten Fettsäuren mit 16 bis 22 C-Atomen enthalten kann und der Veresterungsgrad der gesättigten oder ungesättigten Fettsäuren in der Mischung zwischen 20 und 70 % liegt, als Emulgatoren in kosmetischen und pharmazeutischen Zubereitungen.

WO01/72683 offenbart neuartige ferulyl substituierte oder coumaryl substituierte Acylglycerine erhältlich durch lipase-katalysierte Umesterungen und deren Verwendung als Sonnenschutzmittel mit Breitspektrum-UV-Schutz.

Die Aufgabe der Erfindung bestand daher darin, einen Emulgator bereitzustellen, der vollkommen auf nachwachsenden Rohstoffen basiert (d.h. z.B. dass in der Synthese weder Ethylenoxid, Methanol, Chlor- oder Schwefelderivate Anwendung finden) und der sich darüber hinaus zur Formulierung von O/W Emulsionen (Cremes, Lotionen) mit hervorragender Lagerstabilität und einem angenehm pflegenden Hautgefühl eignet.

<u>Beschreibung der Erfindung</u>

Überraschenderweise wurde gefunden, dass die im Folgenden beschriebenen (Poly-)glycerinpartialesters die gestellte Aufgabe lösen.

Gegenstand der vorliegenden Erfindung sind daher (Poly-)glycerinpartialester mit einer oder mehrerer Carbonsäuren mit 10 bis 24 Kohlenstoffatomen und Resten einer polyfunktionalen Carbonsäure, enthaltend Polyfunktionscarbonsäureester(poly-)glycerinpartialester der allgemeinen Formel (I) wie in Anspruch 1 beschrieben, ein Verfahren zu deren Herstellung, deren Verwendung als Emulgator und Formulierungen, die diese enthalten.

Vorteile der erfindungsgemäßen (Poly-)glycerinpartialester sind, dass sie es ermöglichen, stabile kosmetische und dermatologische O/W Emulsionen herzustellen, die durch die Verwendung organischer Säuren bei pH-Werten von 3,5 bis 5.5 konserviert werden können.
Ein weiterer Vorteil ist, dass die erfindungsgemäßen (Poly-)glycerinpartialester Pigmente oder Festkörper überaus stabil in Emulsionspräparaten halten.

Gegenstand der vorliegenden Erfindung sind (Poly-)glycerinpartialester mit im Mittel (Zahlenmittel) von 0,75 bis 2,25 Säureresten einer oder mehrerer Carbonsäuren mit 10 bis 24, bevorzugt 12 bis 22, besonders bevorzugt 14 bis 18, Kohlenstoffatomen und mit im Mittel (Zahlenmittel) von 0,005 bis 0,5 Resten einer polyfunktionalen Carbonsäure (Polyfunktionscarbonsäure), enthaltend Polyfunktionscarbonsäureester(poly-)glycerinpartialester, mit der Maßgabe, dass nach vollständiger Hydrolyse des (Poly-)glycerinpartialesters ein (Poly-)glycerin erhalten wird, welches eine Homologenverteilung aufweist mit (in Klammern angegeben sind bevorzugte Bereiche):

Glycerin: 0,01 Gew.-% bis 20 Gew.-% (3 Gew.-% bis 12 Gew.-%),
Diglycerine: 0,01 Gew.-% bis 60 Gew.-% (20 Gew.-% bis 40 Gew.-%),
Triglycerine: 0,01 Gew.-% bis 60 Gew.-% (15 Gew.-% bis 35 Gew.-%),
Tetraglycerine: 0,01 Gew.-% bis 30 Gew.-% (5 Gew.-% bis 20 Gew.-%),
Pentaglycerine: 0,01 Gew.-% bis 20 Gew.-% (0,1 Gew.-% bis 15 Gew.-%) und
Oligoglycerine: ad 100 Gew.-%,

wobei sich die angegebenen Gewichtsprozente auf die Gesamtmenge an (Poly-)glycerin beziehen und diese Verteilung mit der GC-Methode wie unten ausgeführt bestimmt wird.

Unter dem Begriff "polyfunktionale Carbonsäuren" oder "Polyfunktionscarbonsäuren" im Sinne der vorliegenden Erfindung sind Carbonsäuren zu verstehen, die mehr als eine Carboxylgruppe aufweisen.

Erfindungsgemäß bevorzugte Polyfunktionscarbonsäure sind die in der EP1683781 genannten Dimer-Fettsäuren, Di- und Tricarbonsäuren, insbesondere Oxalsäure, Fumarsäure, Malonsäure, Bernsteinsäure, Glutarsäure, Adipinsäure, Pimelinsäure, Korksäure, Azelainsäure, Sebacinsäure, Dodecandisäure, wie auch Hydroxydi- bzw. tricarbonsäuren, insbesondere Äpfelsäure, Weinsäure, Tartronsäure, Maleinsäure und Zitronensäure, wie auch aromatische Säuren, insbesondere Phthalsäure, Isophthalsäure oder Terephhtalsäure, wobei Zitronensäure besonders bevorzugt ist.

Somit sind erfindungsgemäß besonders bevorzugte (Poly-)glycerinpartialester solche (Poly-)glycerinpartialester mit im Mittel (Zahlenmittel) von 0,75 bis 2,25 Säureresten einer oder mehrerer Carbonsäuren mit 10 bis 24, bevorzugt 12 bis 22, besonders bevorzugt 14 bis 18, Kohlenstoffatomen und mit im Mittel (Zahlenmittel) von 0,005 bis 0,5 Zitronensäureresten, enthaltend Zitronensäureester(poly-)glycerinpartialester der allgemeinen Formel (I):

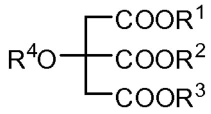 Formel (I),

wobei $R^1$, $R^2$, $R^3$ und $R^4$ unabhängig voneinander gleich oder ungleich ausgewählt sind aus
H oder
ein Rest der allgemeinen Formel (II)

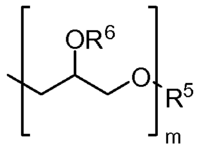 Formel (II),

wobei m größer oder gleich 1 und $R^5$ und $R^6$ unabhängig voneinander gleich oder ungleich ausgewählt sind aus H oder ein Acylrest mit 10 bis 24, bevorzugt 12 bis 22, besonders bevorzugt 14 bis 18, Kohlenstoffatomen,
wobei der Acylrest bestimmt wird durch den Acylrest der mit dem (Poly-)glycerin veresterten Carbonsäure und im Mittel (Zahlenmittel) mindestens einer der Reste $R^5$ oder $R^6$ = H ist, mit der Maßgabe, dass im Mittel (Zahlenmittel) mindestens ein Rest $R^1$, $R^2$ oder $R^3$ ungleich H ist, und mit der Maßgabe, dass nach vollständiger Hydrolyse des (Poly-)glycerinpartialesters ein (Poly-)glycerin erhalten wird, welches eine Homologenverteilung aufweist mit (in Klammern angegeben sind bevorzugte Bereiche):

Glycerin: 0,01 Gew.-% bis 20 Gew.-% (3 Gew.-% bis 12 Gew.-%),
Diglycerine: 0,01 Gew.-% bis 60 Gew.-% (20 Gew.-% bis 40 Gew.-%),
Triglycerine: 0,01 Gew.-% bis 60 Gew.-% (15 Gew.-% bis 35 Gew.-%),
Tetraglycerine: 0,01 Gew.-% bis 30 Gew.-% (5 Gew.-% bis 20 Gew.-%),
Pentaglycerine: 0,01 Gew.-% bis 20 Gew.-% (0,1 Gew.-% bis 15 Gew.-%) und
Oligoglycerine: ad 100 Gew.-%,

wobei sich die angegebenen Gewichtsprozente auf die Gesamtmenge an (Poly-)glycerin beziehen und diese Verteilung mit der GC-Methode wie unten ausgeführt bestimmt wird.

Dem Fachmann ist bewusst, dass Polyglycerin aufgrund seiner polymeren Eigenschaft eine statistische Mischung verschiedener Verbindungen darstellt. Polyglycerin kann ausgebildete Etherbindungen zwischen zwei primären, einer primären und einer sekundären oder zwei sekundären Positionen der Glycerinmonomere aufweisen; ebenfalls sind zyklische Strukturen mit einem oder mehreren Ringen bekannt. Für Details siehe z.B. "Original synthesis of linear, branched and cyclic oligoglycerol standards", Cassel et al., Eur. J. Org. Chem. 2001, 875-896.

Eine geeignete GC Methode zur Bestimmung der Homologenverteilung beinhaltet die Hydrolyse oder Alkoholyse des erfindungsgemäßen (Poly-)glycerinpartialesters, Trennung des Polyglycerins von den entstandenen Säuren und Analyse durch Gaschromatographie.
Hierzu werden 0,6 g des erfindungsgemäßen (Poly-)glycerinpartialesters in 25 ml einer ethanolischen 0,5 M KOH Lösung unter Rückfluss für 30 Minuten gekocht und der pH mit Schwefelsäure auf pH 2-3 eingestellt. Die entstandenen Fettsäuren werden durch dreimalige Extraktion mit jeweils einem Volumen Petrolether abgetrennt. Die vereinigten Extrakte werden durch Evaporation auf ca. 10 ml eingeengt.
Ein 0,5 ml Probe wird in einem Autosamplergefäß mit 0,5 ml MTBE und 1 ml Trimethylanilinium Hydroxide (0.2 M in Methanol) versetzt und mit GC analysiert. Diese wird in einem Gaschromatographen, welcher mit einem split/splitless Injektor, einer Kapillarsäule und einem Flammenionisationdetektor ausgestattet ist, unter folgenden Bedingungen durchgeführt:

| | | |
|---|---|---|
| Injektor | 290 °C, Split 30 ml | |
| Injektionsvolumen: | 1 µl | |
| Säule: | 30 m *0,32 mm HP1 0,25 µm | |
| Trägergas: | Helium, head pressure 70 kPa | |
| Temperaturprogramm: | 80 °C - 300 °C mit 8 °C/min, dann 20 Minuten bei 300 °C konditionieren. | |
| Detektor: | FID bei | 320 °C |
| | Wasserstoff | 35 ml/min |

(fortgesetzt)

| | |
|---|---|
| Luft | 240 ml/min |
| Make Up Gas | 12 ml/min |

Hierdurch werden die Fettsäuren als ihre Methylester nach ihrer Kohlenstoffkettenlänge getrennt. Der relative Gehalt der einzelnen Fettsäuren kann durch Auswertung der Peakflächen bestimmt werden.
Der mit Petrolether extrahierte Rückstand wird mit Bariumhydroxid auf pH 7 bis 8 eingestellt, das ausgefallene Bariumsulfat durch Zentrifugation abgetrennt.
Der Überstand wird abgenommen und der Rückstand wird dreimal mit 20 ml Ethanol extrahiert.
Die vereinigten Überstände werden bei 80 °C und 50 mbar eingeengt, der Rückstand in Pyridin aufgenommen. Ein 0,5 ml Probe wird in einem Autosamplergefäß mit 1 ml N-Methyl-N-trifluoroacetamid versetzt und für 30 Minuten bei 80 °C erhitzt.
Das Polyglycerin wird als sein Trimethylsilyl-Derivat mit GC analysiert, wobei ein Gas-Flüssig-Chromatograph mit einem On-column Injektor und Flammenionisationdetektor unter den folgenden Bedingungen eingesetzt wird:

| | |
|---|---|
| Injektor: | On-column, oven tray |
| Injektionsvolumen: | 0.1 $\mu$l |
| Trägergas: | 3 ml/min Wasserstoff (constant flow) |
| Säule: | SimDist 12 m x 0,32 mm x 0,1 $\mu$m (Varian) |
| Temperaturprogramm: | 65 °C - 365 °C, 10 °C/min; dann 15 Minuten bei 365 °C konditionieren. |
| Detektor (FID): | 375 °C |

Unter diesen Bedingungen wird das Polyglycerin nach dem Polyymerisierungsgrad getrennt; zusätzlich können zyklische von lineraren Isomeren bis zu einem Polymerisierungsgrad von fünf getrennt werden. Die Peakflächen der einzelnen Oligomere werden durch eine Lotrechte an der tiefsten Stelle zwischen den Peaks voneinander getrennt. Da die Auflösung für Oligomere, die einen höheren Polymerisationsgrad als sechs aufweisen, gering ist, werden die Peakflächen für Heptaglycerin und höhere Oligomere zusammengefasst und für die Berechnung des Polydispersitätsindexes als Heptaglycerin betrachtet. Zur Berechnung des Polydispersitätsindexes werden außerdem zyklische und lineare Isomere zusammengefasst.
Der relative Gehalt der einzelnen Oligomere/Isomere kann durch Auswertung der Peakflächen bestimmt werden.
In analoger Weise kann dieses Verfahren auch genutzt werden, um die eingesetzten Rohstoffe, welche zur Herstellung der erfindungsgemäßen Ester eingesetzt werden, zu charakterisieren.

Als Acylreste für $R^5$ und $R^6$ kommen in den erfindungsgemäßen (Poly-)glycerinpartialestern gleiche oder verschiedene Reste in Betracht, wobei es bevorzugt ist, dass es sich in diesem Zusammenhang um unterschiedliche handelt. Dies liegt in der Natur des Herstellungsverfahrens begründet, in dem bevorzugt technische Mischungen von Carbonsäuren, aus denen diese Acylreste hervorgehen, eingesetzt werden. Bevorzugte Acylreste mit 10 bis 24, bevorzugt 12 bis 22, besonders bevorzugt 14 bis 18, Kohlenstoffatomen sind die Acylreste der Säuren Laurinsäure, Tridecansäure, Myristinsäure, Palmitinsäure, Margarinsäure, Stearinsäure, Isostearinsäure, Arachinsäure und Behensäure sowie Mischungen hieraus. Natürlich vorkommende Mischungen sind beispielsweise die Kokosfettsäuren, die als Hauptbestandteil Laurinsäure, daneben noch gesättigte $C_{14}$-$C_{18}$-Fettsäuren und gegebenenfalls gesättigte $C_8$-$C_{10}$-Fettsäuren und ungesättigte Fettsäuren enthalten, sowie Talgfettsäuren, welche im Wesentlichen eine Mischung aus Palmitinsäure und Stearinsäure darstellen und liefern in diesem Zusammenhang die besonders bevorzugten Acylreste, die im Verhältnis Stearinsäure zu Palmitinsäure von 100:0,01 zu 0,01:100 breit variiert werden können. Bevorzugt ist dabei ein Gewichtsverhältnis von 30:70 bis 95:5, besonders ein Verhältnis von 45:55 bis 90:10.
Es kommen auch die Acylreste der monoolefinisch ungesättigten Säuren, beispielsweise Hexadecensäuren, Octadecensäuren, wie Ölsäure (cis-9-Octadecensäure) oder Elaidinsäure (trans-9-Octadecensäure), Eicosensäuren und Docosensäuren, wie Erucasäure (cis-13-Docosensäure) oder Brassidinsäure (trans-13-Docosensäure), wie auch der mehrfach ungesättigte Fettsäuren, beispielsweise Octadecadiensäuren und Octadecatriensäuren, wie Linolsäure und Linolensäure, sowie Mischungen hieraus, in Betracht. In diesem Zusammenhang sind die flüssigen Fettsäuren wie Öl-, Ricinol-, Eruca- und Isostearinsäure, die 18 bis 22 Kohlenstoffatome enthalten, besonders geeignet. Ihre Erstarrungspunkte liegen aufgrund einer Verzweigung oder einer Doppelbindung in der Kohlenwasserstoffkette unter 35 °C. Verwendbar sind weiterhin Fettsäuregemische, die auch wachsartige Komponenten, wie gehärtete Ricinolsäure, enthalten können.

Es ist erfindungsgemäß bevorzugt, dass das nach vollständiger Hydrolyse des erfindungsgemäßen (Poly-)glycerinpartialesters erhaltene (Poly-)glycerin einen mittlere Polymerisationsgrad von 2 bis 6, bevorzugt von 2,5 bis 4,5 und ganz besonders bevorzugt von 3 bis 4 aufweist.

Für die Berechnung wird der mittlere Polymerisationsgrad des Polyglycerins <n> über die Hyroxylzahl (OHV, in mg KOH/g) gemäß der Formel <n> = (112200 - 18*OHV)/(74*OHV - 56100) berechnet.
Geeignete Bestimmungsmethoden zur Ermittlung der Hydroxylzahl sind insbesondere solche gemäß DGF C-V 17 a (53), Ph. Eur. 2.5.3 Method A und DIN 53240.

Es ist vorteilhaft, wenn das nach vollständiger Hydrolyse des erfindungsgemäßen (Poly-)glycerinpartialesters erhaltene (Poly-)glycerin einen Polydispersitätsindex von 0,8 bis 2,5, bevorzugt von 1,0 bis 1,8 aufweist.
Der Polydispersitätsindex lässt such wie folgt berechnen:

$$\sum_i \left| n_i - < n > \right| \cdot x_i \ ,$$

wobei

$n_i$ den Grad der Polymerisierung des einzelnen Oligomeres i,
<n> der mittlere Polymerisationsgrad des Polyglycerins und
$x_i$ der Anteil des Oligomers i im Polyglycerin, bestimmt nach der oben beschriebenen GC Methode
ist.

Vorteilhafte (Poly-)glycerinpartialester gemäß der vorliegenden Erfindung sind dadurch gekennzeichnet, dass nach vollständiger Hydrolyse des (Poly-)glycerinpartialester das molare Verhältnis der erhaltenen Carbonsäuren abgeleitet von $R^5$ und $R^6$ zu (Poly-)glycerin zwischen 2:3 und 4:1, insbesondere zwischen 1:1,2 und 3:1, ganz besonders bevorzugt zwischen 1:1 und 2:1 liegt.
Zur Bestimmung der molaren Verhältnisse lässt sich als Methode die oben beschriebene GC-Methode einsetzen.

Es ist erfindungsgemäß bevorzugt, dass nach vollständiger Hydrolyse des (Poly-)glycerinpartialesters das molare Verhältnis der erhaltenen Polyfunktionscarbonsäure, insbesondere der erhaltenen Zitronensäure zu (Poly-)glycerin zwischen 1:2 und 1:200, insbesondere zwischen 1:5 und 1:150, ganz besonders bevorzugt zwischen 1:10 und 1:100 liegt.
Zur Bestimmung der molaren Verhältnisse lässt sich als Methode die oben beschriebene GC-Methode einsetzen.

Erfindungsgemäß bevorzugte (Poly-)glycerinpartialester sind dadurch gekennzeichnet, dass das Verhältnis von Verseifungszahl (VZ) zu Hydroxylzahl (OHZ) zwischen 1:1,3 und 1:2,6 insbesondere zwischen 1:1,5 und 1:2,4, ganz besonders bevorzugt zwischen 1:1,6 und 1:2,2.
Die Säurezahl (SZ) der erfindungsgemäßen (Poly-)glycerinpartialester beträgt bevorzugt $< 50$, insbesondere $< 10$, ganz besonders bevorzugt $< 5$. Geeignete Bestimmungsmethoden zur Ermittlung der Säurezahl sind insbesondere solche gemäß DGF C-V 2, Ph.Eur. 2.5.1, ISO 3682, ASTM D 974 und DIN EN ISO 2114, geeignete Bestimmungsmethoden zur Ermittlung der Verseifungszahl die DIN EN ISO 3657: 2003-12 und DIN 53401: 1988-06.
Es ist erfindungsgemäß bevorzugt, dass der erfindungsgemäße (Poly-)glycerinpartialester einen Schmelzpunkt von größer 35°C, bevorzugt größer 40°C, insbesondere größer 45°C aufweist.

Erfindungsgemäße (Poly-)glycerinpartialester sind erhältlich nach dem im Folgenden beschriebenen Verfahren; somit ist ein weiterer Gegenstand der Erfindung ein Verfahren zur Herstellung eines (Poly-)glycerinpartialesters enthaltend Polyfunktionscarbonsäureester(poly-)glycerinpartialester umfassend die Verfahrensschritte

A) Bereitstellen eines (Poly-)glycerins aufweisend eine Homologenverteilung mit (in Klammern angegeben sind bevorzugte Bereiche)

Glycerin: 0,01 bis 20 (3 bis 12) Gew.-%
Diglycerine: 0,01 bis 60 (20 bis 40) Gew.-%
Triglycerine: 0,01 bis 60 (15 bis 35) Gew.-%
Tetraglycerine: 0,01 bis 30 (5 bis 20) Gew.-%
Pentaglycerine: 0,01 bis 20 (0,1 bis 10) Gew.-%
Oligoglycerine: ad 100 Gew.-%,

wobei sich die angegebenen Gewichtsprozente auf die Gesamtmenge an (Poly-)glycerin beziehen und diese Verteilung mit der GC-Methode wie oben ausgeführt bestimmt wird,
B) Verestern eines Teils des (Poly-)glycerins mit einer oder mehreren Carbonsäuren mit 10 bis 24, bevorzugt 12 bis 22, besonders bevorzugt 14 bis 18, Kohlenstoffatomen und
C) weiteres Verestern mit einer Polyfunktionscarbonsäure, wobei das molare Verhältnis der in Verfahrensschritt C) eingesetzten Polyfunktionscarbonsäure zu in Verfahrensschritt A) eingesetztem (Poly-)glycerin 1:2 bis 1:200, bevorzugt 1:5 bis 1:150, ganz besonders bevorzugt 1:10 bis 1:100 beträgt.

In dem erfindungsgemäßen Verfahren eingesetzten Polyfunktionscarbonsäure sind bevorzugt ausgesucht aus der Gruppe enthaltend, bevorzugt bestehend aus
die in der EP1683781 genannten Dimer-Fettsäuren, Oxalsäure, Fumarsäure, Malonsäure, Bernsteinsäure, Glutarsäure, Adipinsäure, Pimelinsäure, Korksäure, Azelainsäure, Sebacinsäure, Dodecandisäure, Äpfelsäure, Weinsäure, Tartronsäure, Maleinsäure, Zitronensäure, Phthalsäure, Isophthalsäure und Terephhtalsäure,
wobei Zitronensäure besonders bevorzugt eingesetzt wird.

In einer alternativen Ausführungsform des erfindungsgemäßen Verfahrens kann Verfahrensschritt C) auch vor Verfahrensschritt B) durchgeführt werden.

Das in dem erfindungsgemäßen Verfahren eingesetzte molare Verhältnis der in Verfahrensschritt B) eingesetzten Carbonsäuren zu in Verfahrensschritt A) eingesetztem (Poly-)glycerin beträgt bevorzugt 2:3 bis 4:1, insbesondere 1:1,2 bis 3:1, ganz besonders bevorzugt 1:1 bis 2:1.

Es ist erfindungsgemäß bevorzugt, dass der mittlere Polymerisationsgrad des eingesetzten Polyglycerins in Schritt A) 2 bis 6, bevorzugt 2,5 bis 4,5 und ganz besonders bevorzugt 3 bis 4 beträgt.

Das (Poly-)glycerin für Verfahrensschritt A) kann bereitgestellt werden durch verschiedene Methoden wie beispielsweise Polymerisation von Glycidol (z.B. basenkatalysiert), Polymerisation von Epichlorhydrin (beispielsweise in Gegenwart äquimolarer Mengen einer Base wie NaOH) oder Polykondensation von Glycerin. Erfindungsgemäß bevorzugt ist die Bereitstellung des (Poly-)glycerins durch die Kondensation von Glycerin, insbesondere in Gegenwart katalytischer Mengen einer Base, insbesondere NaOH oder KOH. Geeignete Reaktionsbedingungen sind Temperaturen zwischen 220-260 °C und reduzierter Druck in einem Bereich zwischen 20 bis 800 mbar, insbesondere zwischen 50 und 500 mbar, wodurch eine erleichterte Wasserentfernung möglich ist.

Ein besonders bevorzugtes Verfahren zur Bereitstellung des (Poly-)glycerins mit der benötigten Homologenverteilung, welches über dies hinaus zu einem hohen Polydispersitätsindex und dem bevorzugten Polymerisationsgrad führt, umfasst die Verfahrensschritte

A1) Umsetzung von Glycerin in Gegenwart einer katalytischen Menge an Base, bevorzugt 0,2 bis 5 Gew.% NaOH oder KOH bezogen auf den gesamten Reaktionsansatz, in einem Temperaturbereich von 220-260 °C und in einem Druckbereich zwischen 250 und 1000 mbar, bevorzugt unter destillativem Abtrennen von Wasser, bis die Reaktionsmischung weniger als 70 Gew.-%, bevorzugt weniger als 60 Gew.-% Glycerin bezogen auf den gesamten Reaktionsansatz enthält,
A2) Weitere Umsetzung bei reduziertem Druck in einem Bereich von 20 bis 200 mbar unter destillativem Abtrennen von Wasser und Glycerin , bis die Hydoxylzahl der Reaktionsmischung kleiner 1400, bevorzugt kleiner 1200 beträgt, und optional
A3) Neutralisierung des Katalysators mit einer Säure, bevorzugt einer mineralischen.

In dem Erfindungsgemäßen Verfahren werden die Verfahrensschritte B) und C) unter dem Fachmann bekannten Bedingungen für Veresterungsreaktionen, gegebenenfalls in Anwesenheit eines Katalysators, durchgeführt. Insbesondere wird diese Veresterung unter Entfernung von Wasser aus dem Reaktionsgemisch durchgeführt.
Verfahrensschritt B) wird bevorzugt bei 180-260 °C, besonders bevorzugt 210-250°C durchgeführt, Verfahrensschritt C) bevorzugt bei 100-170 °C, insbesondere bevorzugt bei 120-140 °C.
Der Reaktionsverlauf kann beispielsweise über die Säurezahl des Produktes überwacht werden, so dass es in den Verfahrensschritten B) und C) bevorzugt ist, diese so lange durchzuführen, bis die gewünschte Säurezahl erreicht wird.
In Schritt C wird die eingesetzte Polyfunktionscarbonsäure in der Regel nicht vollständig verestert, sondern so umgesetzt, dass die erfindungsgemäßen (Poly-)glycerin-partialester teilweise noch freie Carboxylgruppen enthalten.

Die in dem erfindungsgemäßen Verfahren in Verfahrensschritt B) eingesetzten Carbonsäuren sind bevorzugt solche, die oben als bevorzugte die Acylreste $R^5$ und $R^6$ in erfindungsgemäßen (Poly-)glycerinpartialestern liefernde Carbonsäuren genannt wurden. Insbesondere werden in diesem Zusammenhang die oben genannten Talgfettsäuren mit beschriebenen Verhältnissen von Palmitin- und Stearinsäure eingesetzt.

Erfindungsgemäße (Poly-)glycerinpartialester und (Poly-)glycerinpartialester erhältlich bzw. erhalten nach dem erfindungsgemäßen Verfahren eignen sich hervorragend zur Verwendung als leistungsstarker O/W Emulgator, der ausschließlich auf nachwachsenden Rohstoffen basiert und eine große Formulierungsflexibilität aufweist, insbesondere in kosmetischen Formulierungen.

Somit sind Emulgatoren enthaltend erfindungsgemäße (Poly-)glycerinpartialester oder (Poly-)glycerinpartialester erhältlich bzw. erhalten nach dem erfindungsgemäßen Verfahren Gegenstand der vorliegenden Erfindung. Als Emulgator ist im Rahmen dieser Erfindung ein Emulgator zu verstehen, der mindestens aus einem erfindungsgemäßen (Poly-)glycerinpartialester oder (Poly-)glycerinpartialester erhältlich bzw. erhalten nach dem erfindungsgemäßen Verfahren und gegebenenfalls mindestens einem Co-Emulgator besteht, wobei die Anwesenheit eines Co-Emulgators

bevorzugt ist.

Ebenso eignen sich erfindungsgemäße (Poly-)glycerinpartialester und (Poly-)glycerinpartialester erhältlich bzw. erhalten nach dem erfindungsgemäßen Verfahren hervorragend zur Verwendung zur Herstellung von kosmetischen oder pharmazeutischen Formulierungen, insbesondere zur Herstellung kosmetischer Cremes und Lotionen.
Unter Cremes und Lotionen wird in diesem Zusammenhang kosmetische O/W Emulsionen mit streichfähig-pastöser bzw. fließfähiger Konsistenz verstanden.
Allgemein können die erfindungsgemäßen (Poly-)glycerinpartialester beispielsweise in Pflegecremes und -lotionen für Gesicht, Körper und Hände, in Sonnenschutzemulsionen, in Schminken, in Aersolen, Rollons, Pumpsprays, Stiften z.B. im AP/Deo-Bereich, in Babypflegeprodukten, in Intimpflege-, Fußpflege-, Haarpflege-, Nagelpflege, Zahnpflege- oder Mundpflegeprodukten sowie in dermatologischen Salben eingesetzt werden.

Somit sind ebenfalls kosmetischen oder pharmazeutischen Formulierungen, insbesondere O/W Formulierungen, enthaltend erfindungsgemäße (Poly-)glycerinpartialester oder (Poly-)glycerinpartialester erhältlich bzw. erhalten nach dem erfindungsgemäßen Verfahren Gegenstand der vorliegenden Erfindung. Erfindungsgemäß bevorzugte Formulierungen stellen Sonnschutzpräparate und O/W Make-Up Formulierungen dar.

Erfindungsgemäß bevorzugte Formulierungen, enthalten den erfindungsgemäßen (Poly-)glycerinpartialester oder (Poly-)glycerinpartialester erhältlich bzw. erhalten nach dem erfindungsgemäßen Verfahren in Mengen von 0.01 bis 10 Gew.%, bevorzugt 0.05 bis 8 Gew.% und besonders bevorzugt 0.1 bis 5 Gew.% bezogen auf die Gesamtformulierung.

Die erfindungsgemäßen Formulierungen können z.B. mindestens eine zusätzliche Komponente enthalten, ausgewählt aus der Gruppe der

Emollients,
Co-Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.
Bezüglich weiterer fakultativer Komponenten sowie deren eingesetzten Mengen dieser Komponenten wird ausdrücklich auf die dem Fachmann bekannten einschlägigen Handbücher, z. B. K. Schrader, "Grundlagen und Rezepturen der Kosemtika", 2. Auflage, Seite 329 bis 341, Hüthig Buch Verlag Heidelberg, verwiesen.
Die Mengen der jeweiligen Zusätze richten sich nach der beabsichtigten Verwendung.
Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Da die erfindungsgemäßen (Poly-)glycerinpartialester und Emulgatoren Pigmente oder Festkörper überaus stabil in Emulsionspräparaten halten können, sind Fest- und Füllstoffe, insbesondere Partikel und Additive, die zur Erzielung eines spezifischen Hautgefühls eingesetzt werden, wie z.B. Siliconelastomere, PMMA-Partikel, PE-Partikel, PS-Partikel, Nylon-Partikel, Bornitrid, Stärke, Mica und Talkum, eine bevorzugte zusätzliche Komponente.

Erfindungsgemäß bevorzugt sind insbesondere Formulierungen, die als Konservierungsstoffe organische Säuren, insbesondere Sorbin-, Benzoe- und/oder Dehydroacetsäure, insbesondere in einem Bereich von 0,01 bis 1,0 Gew.% bezogen auf die Gesamtformulierung, enthalten.

Des Weiteren weisen erfindungsgemäß bevorzugte Formulierungen einen pH-Wert von 3,5 bis 5,5, insbesondere von 4,0 bis 5,0 auf.

Es wurde gefunden, dass sich die erfindungsgemäßen (Poly-)glycerinpartialester hervorragend dazu eignen, die Wasserfestigkeit von erfindungsgemäßen Formulierungen zu erhöhen. Dies ist insbesondere für Sonnenschutzpräparate sowie auch für O/W Make-Up Formulierungen vorteilhaft. Somit ist ein weiterer Gegenstand der Erfindung die Verwendung der erfindungsgemäßen (Poly-)glycerinpartialester oder (Poly-)glycerinpartialester erhältlich bzw. erhalten nach dem erfindungsgemäßen Verfahren sowie die erfindungsgemäßen Emulgatoren zur Verbesserung der Wasserfestigkeit von kosmetischen oder pharmazeutischen Formulierungen, insbesondere von Sonnenschutzpräparaten, wobei diese bevorzugt Pigmente oder Festkörper, insbesondere Titanoxid, enthalten.

Unter Wasserfestigkeit wird in diesem Zusammenhang die Verhinderung leichter Ablösbarkeit der Formulierung von einer Oberfläche, insbesondere von Haut, durch Kontakt mit Wasser verstanden. Die Wasserfestigkeit kann durch einen einfachen *in-vitro* Test beschrieben wie in Int. J. Cosm. Sci., 2007, 29, 451-460 bestimmt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Alle angegebenen Prozent (%) sind wenn nicht anders angegeben Massenprozent.

Beispiele:

*Beispiel 1: Emulgator 1:*

90 g Polyglycerin, mit einer mittleren OH-Zahl von 1190 mg KOH/g, werden mit 210 g Stearinsäure bei 240°C unter Einleitung von Stickstoff umgesetzt. Das Reaktionswasser wird abdestilliert. Bei einer Säurezahl von < 2 mg KOH/g kühlt man den Polyglycerinester auf 130°C ab, gibt 40 g Zitronensäure zu und reduziert den Druck auf 50 mbar. Das bei dieser Reaktion entstehende Wasser wird wieder abgetrennt. Bei einer Säurezahl < 50 wird das Vakuum mit Stickstoff gebrochen und das Produkt abgefüllt.

| | |
|---|---|
| OH-Zahl: | 237 mg KOH/g |
| SZ: | 45 mg KOH/g |
| Verseifungszahl: | 189 mg KOH /g |

*Beispiel 2: Emulgator 2:*

137,2 g Polyglycerin, mit einer mittleren OH-Zahl von 1120 mg KOH/g, werden mit 254,8 g Stearinsäure, bei 240°C unter Einleitung von Stickstoff umgesetzt. Das Reaktionswasser wird abdestilliert. Bei einer Säurezahl von < 2 mg KOH/g kühlt man den Polyglycerinester auf 130°C ab, gibt 8 g Zitronensäure zu und reduziert den Druck auf 50 mbar. Das bei der Reaktion entstehende Wasser wird wieder abgetrennt.

Bei einer Säurezahl <1 wird das Vakuum mit Stickstoff gebrochen und das Produkt abgefüllt.

| | |
|---|---|
| OH-Zahl: | 235 mg KOH/g |
| SZ: | 2,3 mg KOH/g |
| Verseifungszahl: | 145 mg KOH/g |

*Beispiel 3: Emulgator 3:*

152,8g Polyglycerin, mit einer mittleren OH-Zahl von 1000 mg KOH/g, werden mit 229,2 g Stearinsäure bei 240°C unter Einleitung von Stickstoff umgesetzt. Das Reaktionswasser wird abdestilliert. Bei einer Säurezahl von < 2 mg KOH/g kühlt man den Polyglycerinester auf 130°C ab, gibt 18 g Zitronensäure zu und reduziert den Druck auf 50 mbar. Das bei der Reaktion entstehende Wasser wird wieder abgetrennt. Bei einer Säurezahl < 3 mg KOH/g wird das Vakuum mit Stickstoff gebrochen und das Produkt abgefüllt.

| | |
|---|---|
| OH-Zahl: | 241 mg KOH/g |
| SZ: | 2,6 mg KOH/g |
| Verseifungszahl: | 140 mg KOH /g |

*Beispiel 4: Emulgator 4:*

114,6 g Polyglycerin, mit einer mittleren OH-Zahl von 1120 mg KOH/g, werden mit 267,4 g Palmitinsäure bei 240°C unter Einleitung von Stickstoff umgesetzt. Das Reaktionswasser wird abdestilliert. Bei einer Säurezahl von < 2 mg KOH/g kühlt man den Polyglycerinester auf 130°C ab, gibt 18 g Zitronensäure zu und reduziert den Druck auf 50 mbar. Das bei der Reaktion entstehende Wasser wird wieder abgetrennt. Bei einer Säurezahl < 3 mg KOH/g wird das Vakuum mit Stickstoff gebrochen und das Produkt abgefüllt.

| | |
|---|---|
| OH-Zahl: | 189 mg KOH/g |
| SZ: | 2,8 mg KOH/g |
| Verseifungszahl: | 148 mg KOH /g |

*Beispiel 5: Emulgator 5:*

133,7 g Polyglycerin, mit einer mittleren OH-Zahl von 1000 mg KOH/g, werden mit 248,3 g Behensäure bei 240°C unter Einleitung von Stickstoff umgesetzt. Das Reaktionswasser wird abdestilliert. Bei einer Säurezahl von < 2 mg KOH/g kühlt man den Polyglycerinester auf 130°C ab, gibt 18 g Zitronensäure zu und reduziert den Druck auf 50 mbar. Das bei der Reaktion entstehende Wasser wird wieder abgetrennt. Bei einer Säurezahl < 3 mg KOH/g wird das Vakuum mit Stickstoff gebrochen und das Produkt abgefüllt.

| | |
|---|---|
| OH-Zahl: | 287,9 mg KOH/g |
| SZ: | 2,8 mg KOH/g |
| Verseifungszahl: | 111 mg KOH /g |

*Anwendungsbeispiele:*

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.
Die Herstellung der Emulsionen erfolgte daher typischerweise so, dass Öl- und Wasserphase auf 70 - 75°C erwärmt wurden. Anschließend wurde entweder die Ölphase in die Wassereingerührt oder Öl- und Wasserphase ohne Rühren zusammengegeben. Anschließend wurde mit einem geeigneten Homogenisator (z.B. Ultrathurrax) für ca. 1-2 Minuten homogenisiert.
Stabilisierende Polymere (z.B. Carbomere) werden bevorzugt als Öldispersion bei Temperaturen von 50 - 60°C in die Emulsion eingerührt. Anschließend wird kurz homogenisiert. Zugabe weiterer Inhaltsstoffe (z.B. Konservierungsmittel, Wirkstoffe) erfolgte bevorzugt bei 40°C. Falls die Rezepturen mit organischen Säuren konserviert wurden, wurde der pH-Wert der Emulsionen auf ca. 5 eingestellt.

1) Differenzierung der Performance gegen den Stand der Technik

Diese Versuche sollen zeigen, dass die erfindungsgemäßen Polyglycerinpartialester Vorteile in Bezug auf Emulsionsstabilität aufweisen. Als Repräsentanten für vollständig auf natürlichen Rohstoffen basierende O/W Emulgator wurden dabei Glyceryl Stearate Citrate, Polygylceryl-3 Distearate und eine gängige Kombination von Sorbitan Stearate und Sucrose Cocoate gewählt.
Zur Überprüfung der Lagerstabilität der Emulsionen wurden diese drei Monate bei Raumtemperatur, 40°C und 45°C gelagert. Zur Überprüfung der Kältestabilität wurde außerdem ein Monat lang bei -5°C gelagert und drei Gefrier-Tau-Zylken von 25°C/-15°C/25°C durchgeführt. Deutlich Veränderungen im Erscheinungsbild oder der Konsistenz sowie insbesondere Öl- oder Wasserabscheidungen wurden als Kriterien für Instabilität gewichtet.
Um einen fairen Vergleich zu gewährleisten, wurde die Emulgatormenge und die dem entsprechende Konsistenzgebermenge (Stearyl Alcohol, Glyceryl Stearate) jeweils für optimal an den entsprechenden Emulgatortyp angepasst. Der Gesamtölphasengehalt wurde stets auf 25,0 % eingestellt. Somit konnte sichergestellt werden, dass die erfindungsgemäßen Beispiele eine vergleichbare Ausgangskonsistenz hatten wie die Emulsionen mit den Vergleichsemulgatoren.
Vergleich in Cremerezeptur:

| Beispiele | 1 | V1 | V2 | V3 |
|---|---|---|---|---|
| Emulgator 2 | 3,0% | | | |

(fortgesetzt)

| Beispiele | 1 | V1 | V2 | V3 |
|---|---|---|---|---|
| Glyceryl Stearate Citrate 1) | | 1,50% | | |
| Polyglyceryl-3 Distearate 2) | | | 3,00% | |
| Sorbitan Stearate; Sucrose Cocoate 3) | | | | 4,00% |
| Stearyl Alcohol | 1,0% | 6,0% | 1,0% | 2,5% |
| Glyceryl Stearate | 2,0% | | 2,0% | 2,5% |
| Isopropyl Palmitate | 5,0% | 5,0% | 5,0% | 4,5% |
| Caprylic/Capric Triglyceride | 9,0% | 7,5% | 9,0% | 7,0% |
| Avocadoöl | 5,0% | 5,0% | 5,0% | 4,5% |
| Glycerin | 3,0% | 3,0% | 3,0% | 3,0% |
| Demineralized Water | ad 100% | ad 100% | ad 100% | ad 100% |
| Benzyl Alcohol, Benzoic Acid, Sorbic Acid 4) | 1,0% | 1,0% | 1,0% | 1,0% |
| NaOH (5% Lösung) (pH-Wert-Einstellung auf 5,0) | q.s. | q.s. | q.s. | q.s. |
| | | | | |
| Konsistenz nach Herstellung | Pastös, cremeartig | Pastös, cremeartig | Pastös, cremeartig | Pastös, cremeartig |
| Stabilität | Stabil | Wasserseparation | Wasserseparation | Wasserseparation |
| | | nach 2 Monaten 45°C; pH-Abfall auf 4,5 | nach 1 Woche bei 40°C und 45°C | nach 1 Monat bei RT und 40°C und nach 1 Woche bei 45°C |
| 1) AXOL® C 62 (Evonik Goldschmidt)<br>2) Cremophor® GS 32 (BASF)<br>3) Arlatone® 2121 (Croda)<br>4) Rokonsal® BSB-N (ISP) | | | | |

Während die Formulierung mit dem erfindungsgemäßen Emulgator 2 zu einer lagerstabilen Formulierung führt, zeigen die Cremes mit den Vergleichsemulgatoren deutliche Schwächen in der Lagerstabilität.

Ein weiterer Vergleich eines erfindungsgemäßen (Poly-)glycerinpartialesters gegen Polyglyceryl-3 Distearate (V4) und die Kombination Sorbitan Stearate und Sucrose Stearate (V5) wurde in einer O/W Lotion durchgeführt.

| Beispiele | 2 | V4 | V5 |
|---|---|---|---|
| Emulgator 2 | 3,0% | | |
| Polyglyceryl-3 Distearate 2) | | 3,0% | |
| Sorbitan Stearate; Sucrose Cocoate 3) | | | 3,0% |
| Isopropyl Palmitate | 2,5% | 2,5% | 2,5% |
| Caprylic/Capric Triglyceride | 4,5% | 4,5% | 4,5% |

(fortgesetzt)

| Beispiele | 2 | V4 | V5 |
|---|---|---|---|
| Avocadoöl | 5,0% | 5,0% | 5,0% |
| Glycerin | 3,0% | 3,0% | 3,0% |
| Xanthan Gum | 0,5% | 0,5% | 0,5% |
| Demineralized Water | ad 100% | ad 100% | ad 100% |
| Benzyl Alcohol, Benzoic Acid, Sorbic Acid 4) | 1,0% | 1,0% | 1,0% |
| NaOH (5% so-lution) (pH-Wert-Einstellung auf 5,0) | q.s. | q.s. | q.s. |
| Konsistenz nach Herstellung | fließfähig | fließfähig | fließfähig |
| Stabilität | Stabil | Starke Wasserseparation nach 1 Monat bei 45°C | Emulsion trennt sich nach 1 Monat bei 45°C komplett auf |

Hier zeigt sich ebenfalls, dass die Verwendung des erfindungsgemäßen (Poly-)glycerinpartialester im Vergleich zu den Vergleichsemulgatoren zu einer deutlich verbesserten Wärmestabilität führt.

*Weitere Emulsionsbeispiele:*

Diese Beispiele sollen zeigen, dass die erfindungsgemäßen (Poly-)glycerinpartialester in einer Vielzahl kosmetischer Formulierungen eingesetzt werden können.
Es ist darüber hinaus mit Hilfe der erfindungsgemäßen (Poly-)glycerinpartialester möglich, Pigmente oder Festkörper stabil in Emulsionspräparate einzuarbeiten.
Weiterhin zeigen die Beispiele die gute Kompatibilität mit typischen Coemulgatoren, Ölen, Verdickern und Stabilisatoren.

O/W Lotionen

| Beispiele | 3 | 4 | 5 | 6 | 7 |
|---|---|---|---|---|---|
| Emulgator 2 | 3,0% | | 3,0% | | |
| Emulgator 3 | | 2,0% | | | 3,0% |
| Emulgator 4 | | | | 3,0% | |
| Isopropyl Palmitate | 2,5% | | 3,0% | | |
| Caprylic/Capric Triglyceride | 4,5% | | 7,0% | 5,0% | |
| Mandelöl | 5,0% | | | | |
| Avocadoöl | | | 5,0% | | |
| Diethylhexyl Carbonate | | | | | 7,0% |
| Ethylhexyl Palmitate | | 7,3% | 6,5% | 5,8% | 5,8% |
| Mineral Oil | | 6,5% | | | |
| PPG-15 Stearylether | | | | 2,0% | |
| Ceramide 3 | | | 0,2% | | |
| Salicyloyl Phytosphingosine | | | | 0,2% | |
| Cetyl Ricinoleate | | | 2,0% | | |
| Dimethicone/Vinyl Dimethicone Crosspolymer | | | | 2,0 % | |
| Glycerin | 5,0% | 3,0% | 3,0% | 3,0% | 3,0% |
| Hydrolyzed Hyaluronic Acid | | | | | 0,2% |

(fortgesetzt)

| Beispiele | 3 | 4 | 5 | 6 | 7 |
|---|---|---|---|---|---|
| Xanthan Gum | 0,5% | | 0,5% | | |
| Carbomer | | 0,2% | | 0,2% | 0,2% |
| Demineralized Water | ad 100% | ad 100% | ad 100% | ad 100% | ad 100% |
| Benzyl Alcohol, Benzoic Acid, Sorbic Acid 4) | 1,0% | | 1,0% | | |
| NaOH (5% solution) (pH-Wert-Einstel-lung auf 5,0) | q.s. | | q.s. | | |
| Phenoxyethanol, Ethylhexylglycerin 5) | | 0,8% | | | |
| Methylisothiazolinone, Methylparaben, Ethylparaben; Dipropylene Glycol 6) | | | | 0,8% | 0,8% |
| 5) Euxyl® PE 9010 (Schülke)<br>6) Microcare® MEM (Thor) | | | | | |

O/W Cremes

| Beispiele | 8 | 9 | 10 | 11 | 12 |
|---|---|---|---|---|---|
| Emulgator 2 | 3,0% | | 2,0% | | 2,0% |
| Emulgator 3 | | 3,0% | | 2,8% | |
| Glyceryl Stearate | 2,0% | 2,0% | 2,0% | 2,0% | 3,5% |
| Stearyl Alcohol | 1,0% | 2,0% | 1,0% | 2,0% | 1,5% |
| Distearyldimonium Chloride | | | 1,0% | | |
| Sodium Cetearyl Sulfate | | | | 0,2% | |
| Bis-PEG/PPG-20/5 PEG/PPG-20/5 Dimethicone, Methoxy PEG/PPG-25/4 Dimethicone; Caprylic/ Capric Triglyceride 8) | | | | | 1,0% |
| Isopropyl Palmitate | 10,3% | | | 10,0% | |
| Caprylic/Capric Triglyceride | | 7,5% | 10,0% | 5,0% | |
| Mandelöl | | | | 5,0% | |
| Avocadoöl | | 5,0% | | | |
| Diethylhexyl Carbonate | 9,5% | | | | 8,5% |
| Ethylhexyl Palmitate | | | 9,0% | | 5,0% |
| Decyl Cocoate | | 7,0% | | | |
| Dimethicone | | | | | 2,0% |
| Cetyl Ricinoleate | | | | | 1,0% |
| Glycerin | 3,0% | 3,0% | 6,0% | 3,0% | |
| Tetrapeptide-17, Glycerin, Butylene Glycol, Aqua 7) | 2,5% | | | | |
| Curcuma Longa (turmeric root extract) | | 0,5% | | | |
| Carbomer | 0,2% | | | | |
| Demineralized Water | ad 100% | ad 100% | Ad 100% | ad 100% | Ad 100% |
| Benzyl Alcohol, Benzoic Acid, Sorbic Acid 4) | | 1,0% | | 1,0% | |
| NaOH (5% solution) (pH-Wert-Einstel-lung auf 5,0) | | q.s. | | q.s. | |

(fortgesetzt)

| Beispiele | 8 | 9 | 10 | 11 | 12 |
|---|---|---|---|---|---|
| Methylisothiazolinone, Methylparaben, Ethylparaben; Dipropylene Glycol 6) | 0,8% | | | | 0,8% |
| Phenoxyethanol, Ethylhexylglycerin 5) | | | 0,7% | | |
| 7) TEGO® Pep 4-17 (Evonik Goldschmidt)<br>8) ABIL® Care XL 80 (Evonik Goldschmidt) | | | | | |

Sonnenschutzlotion:

| Beispiel | 13 |
|---|---|
| Emulgator 2 | 3,0% |
| Glyceryl Stearate | 0,5% |
| Stearyl Alcohol | 0,5% |
| Diethylhexyl Carbonate | 3,0% |
| Caprylic/Capric Triglyceride | 2,0% |
| C12-15 Alkyl Benzoate | 5,0% |
| Octocrylene | 2,0% |
| Bis-Ethylhexyloxyphenol Methoxyphenyl Triazine | 3,0% |
| Polysilicone-15 | 2,0% |
| Titanium Dioxide; Trimethoxycaprylylsilane | 5,0% |
| Glycerin | 3,0% |
| Xanthan Gum | 0,2% |
| Carbomer | 0,3% |
| Demineralized Water | ad 100% |
| NaOH (5% solution) (pH-Wert-Einstellung auf 6,0) | q.s. |
| Methylisothiazoli-none, Methylpara-ben, Ethylparaben; Dipropylene Glycol [6)] | 0,8% |

Selbstbräunungslotion:

| Beispiel | 13 |
|---|---|
| Emulgator 3 | 3,0% |
| Ceteareth-25 | 0,5% |
| Glyceryl Stearate | 2,5% |
| Stearyl Alcohol | 1,0% |
| Isopropyl Palmitate | 3,0% |
| Caprylic/Capric Triglyceride | 3,0% |
| Mineral Oil | 7,0% |
| Jojoba Oil | 3,0% |
| Glycerin | 3,0% |
| Dihydroxyacetone | 5,0% |
| Demineralized Water | ad 100% |

(fortgesetzt)

| Beispiel | 13 |
|---|---|
| Citric Acid (10% solution) (pH-Wert-Einstellung auf 4,0) | q.s. |
| Methylisothiazolinone, Methylparaben, Ethylparaben; Dipropylene Glycol [6)] | 0,8% |

Haarcreme mit UV-Schutz:

| Beispiel | 13 |
|---|---|
| Emulgator 5 | 3,0% |
| Caprylic/Capric Triglyceride | 4,0% |
| Aprikosenkernöl | 2,5% |
| Mandelöl | 2,5% |
| Cetearyl Ricinoleate | 1,0% |
| Isopropyl Myristate | 2,0% |
| Cetearyl Alcohol | 1,0% |
| Glyceryl Stearate | 1,5% |
| Glycerin | 3,0% |
| Ethylhexyl Methoxycinnamate | 2,0% |
| Polysilicone-19 | 0,5% |
| Silicone Quaternium-22 | 0,2% |
| Demineralized Water | ad 100% |
| Methylisothiazolinone, Methylparaben, Ethylparaben; Dipropylene Glycol [6)] | 0,8% |

PEG-freies AP/Deo Roll-on:

| Beispiel | 14 |
|---|---|
| Emulgator 2 | 2,5% |
| Caprylic/capric triglyceride | 2,0% |
| Diethylhexyl Carbonate | 2,5% |
| PPG-14 Butylether | 2,5% |
| Polyglyceryl-3 Caprylate | 1,0% |
| Palmitamidopropyltrimonium Chloride | 1,0% |
| Demineralized water | ad 100% |
| Hydroxyethyl Cellulose | 1,0% |
| Silicone quaternium-22 | 0,2% |
| Parfum | q.s. |
| Methylisothiazolinone, methylparaben, ethylparaben; dipropylene glycol [6)] | 0,8% |

## Patentansprüche

1. (Poly-)glycerinpartialester mit im Mittel (Zahlenmittel) von 0,75 bis 2,25 Säureresten einer oder mehrerer Carbonsäuren mit 10 bis 24 Kohlenstoffatomen und mit im Mittel (Zahlenmittel) von 0,005 bis 0,5 Resten einer polyfunktionalen Carbonsäure, insbesondere Dimer-Fettsäuren, Oxalsäure, Fumarsäure, Malonsäure, Bernsteinsäure, Glutarsäure, Adipinsäure, Pimelinsäure, Korksäure, Azelainsäure, Sebacinsäure, Dodecandisäure, Äpfelsäure, Weinsäure, Tartronsäure, Maleinsäure, Zitronensäure, Phthalsäure, Isophthalsäure und Terephhtalsäure,
enthaltend Polyfunktionscarbonsäureester(poly-)glycerinpartialester mit der Maßgabe, dass nach vollständiger Hydrolyse des (Poly-)glycerinpartialesters ein (Poly-)glycerin erhalten wird, welches eine Homologenverteilung aufweist mit:

Glycerin: 0,01 Gew.-% bis 20 Gew.-%,
Diglycerine: 0,01 Gew.-% bis 60 Gew.-%,
Triglycerine: 0,01 Gew.-% bis 60 Gew.-%,
Tetraglycerine: 0,01 Gew.-% bis 30 Gew.-%,
Pentaglycerine: 0,01 Gew.-% bis 20 Gew.-% und
Oligoglycerine: ad 100 Gew.-%.

**2.** (Poly-)glycerinpartialester gemäß Anspruch 1, **dadurch gekennzeichnet, dass** die polyfunktionale Carbonsäure Zitronensäure darstellt und Zitronensäureester(poly-)glycerinpartialester der allgemeinen Formel (I) enthält:

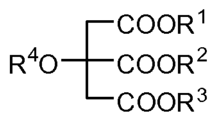 Formel (I),

wobei $R^1$, $R^2$, $R^3$ und $R^4$ unabhängig voneinander gleich oder ungleich ausgewählt sind aus

H oder
ein Rest der allgemeinen Formel (II)

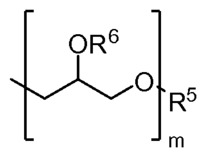 Formel (II),

wobei m größer oder gleich 1 und $R^5$ und $R^6$ unabhängig voneinander gleich oder ungleich ausgewählt sind aus
H oder
ein Acylrest mit 10 bis 24 Kohlenstoffatomen, wobei
der Acylrest bestimmt wird durch den Acylrest der
mit dem (Poly-)glycerin veresterten Carbonsäure

und im Mittel (Zahlenmittel) mindestens einer der Reste $R^5$ oder $R^6$ = H ist, mit der Maßgabe, dass im Mittel (Zahlenmittel) mindestens ein Rest $R^1$, $R^2$ oder $R^3$ ungleich H ist.

**3.** (Poly-)glycerinpartialester gemäß Anspruch 1 oder 2, **dadurch gekennzeichnet, dass** nach vollständiger Hydrolyse des Partialesters das molare Verhältnis der erhaltenen Carbonsäuren abgeleitet von $R^5$ und $R^6$ zu (Poly-)glycerin zwischen 2:3 und 4:1 liegt.

**4.** (Poly-)glycerinpartialester gemäß mindestens einem der Ansprüche 1 bis 3, **dadurch gekennzeichnet, dass** das Verhältnis von Verseifungszahl zu Hydroxylzahl zwischen 1:1,3 und 1:2,6 liegt.

**5.** (Poly-)glycerinpartialester gemäß mindestens einem der Ansprüche 1 bis 4, **dadurch gekennzeichnet, dass** die Säurezahl kleiner 50 beträgt.

**6.** (Poly-)glycerinpartialester gemäß mindestens einem der Ansprüche 1 bis 5, **dadurch gekennzeichnet, dass** er einen Schmelzpunkt von größer 35°C aufweist.

**7.** (Poly-)glycerinpartialester gemäß mindestens einem der Ansprüche 1 bis 6, **dadurch gekennzeichnet, dass** der $R^5$ und $R^6$ bestimmende Acylrest, sich aus einer Mischung von Stearinsäure und Palmitinsäure im Gewichtsverhältnis von 30:70 bis 95:5 ergibt.

**8.** Verfahren zur Herstellung eines (Poly-)glycerinpartialesters umfassend die Verfahrensschritte

A) Bereitstellen eines (Poly-)glycerins aufweisend eine Homologenverteilung mit

Glycerin: 0,01 bis 20 Gew.-%
Diglycerine: 0,01 bis 60 Gew.-%
Triglycerine: 0,01 bis 60 Gew.-%
Tetraglycerine: 0,01 bis 30 Gew.-%
Pentaglycerine: 0,01 bis 20 Gew.-%
Oligoglycerine: ad 100 Gew.-%.

B) Verestern eines Teils des (Poly-)glycerins mit einer oder mehreren Carbonsäure mit 10 bis 24 Kohlenstoffatomen und
C) weiteres Verestern mit einer Polyfunktionscarbonsäure,

wobei das molare Verhältnis der in Verfahrensschritt C) eingesetzten Polyfunktionscarbonsäure zu in Verfahrensschritt A) eingesetztem (Poly-)glycerin 1:2 bis 1:200 beträgt.

**9.** Verfahren gemäß Anspruch 8, **dadurch gekennzeichnet, dass** das eingesetzte molare Verhältnis von der einen oder mehreren Carbonsäuren zu (Poly-)glycerin 2:3 bis 4:1 beträgt.

**10.** Verfahren gemäß Anspruch 8 oder 9, **dadurch gekennzeichnet, dass** die Polyfunktionscarbonsäure Zitronensäure und die Carbonsäure eine Mischung aus Stearinsäure und Palmitinsäure im Gewichtsverhältnis von 30:70 bis 95:5 ist.

**11.** Verwendung des (Poly-)glycerinpartialesters gemäß mindestens einem der Ansprüche 1 bis 7 oder des (Poly-)glycerinpartialesters erhältlich nach einem Verfahren gemäß mindestens einem der Ansprüche 8 bis 10 als Emulgator.

**12.** Verwendung des (Poly-)glycerinpartialesters gemäß mindestens einem der Ansprüche 1 bis 7 oder des (Poly-)glycerinpartialesters erhältlich nach einem Verfahren gemäß mindestens einem der Ansprüche 8 bis 10 zur Herstellung von kosmetischen oder pharmazeutischen Formulierungen.

**13.** Emulgator enthaltend (Poly-)glycerinpartialester gemäß mindestens einem der Ansprüche 1 bis 7 oder (Poly-)glycerinpartialester erhältlich nach einem Verfahren gemäß mindestens einem der Ansprüche 8 bis 10.

**14.** Verwendung des (Poly-)glycerinpartialesters gemäß mindestens einem der Ansprüche 1 bis 7 oder des (Poly-)glycerinpartialesters erhältlich nach einem Verfahren gemäß mindestens einem der Ansprüche 8 bis 10 oder eines Emulgators gemäß Anspruch 13 zur Verbesserung der Wasserfestigkeit von kosmetischen oder pharmazeutischen Formulierungen

**15.** Kosmetische oder pharmazeutische Formulierung enthaltend (Poly-)glycerinpartialester gemäß mindestens einem der Ansprüche 1 bis 7 oder (Poly-)glycerinpartialester erhältlich nach einem Verfahren gemäß mindestens einem der Ansprüche 8 bis 10.

**16.** Kosmetische oder pharmazeutische Formulierung gemäß Anspruch 14, **dadurch gekennzeichnet, dass** der (Poly-)glycerinpartialester in Mengen von 0.01 bis 10 Gew.% enthalten ist.

**17.** Kosmetische oder pharmazeutische Formulierung gemäß Anspruch 14 oder 15, **dadurch gekennzeichnet, dass** als Konservierungsstoff organische Säuren in einem Bereich von 0,01 bis 1,0 Gew.% enthalten sind.

**18.** Kosmetische oder pharmazeutische Formulierung gemäß mindestens einem der Ansprüche 14 bis 16, **dadurch gekennzeichnet, dass** sie einen pH-Wert von 3,5 bis 5,5 aufweisen.

## Claims

**1.** (Poly-)glycerol partial ester with, on average (number-average), from 0.75 to 2.25 acid radicals of one or more carboxylic acids having 10 to 24 carbon atoms and with, on average (number-average), from 0.005 to 0.5 radicals of a polyfunctional carboxylic acid, in particular dimer fatty acids, oxalic acid, fumaric acid, malonic acid, succinic

acid, glutaric acid, adipic acid, pimelic acid, suberic acid, azelaic acid, sebacic acid, dodecanedioic acid, malic acid, tartaric acid, tartronic acid, maleic acid, citric acid, phthalic acid, isophthalic acid and terephthalic acid, comprising polyfunctional carboxylic acid ester (poly-)glycerol partial ester with the proviso that, following complete hydrolysis of the (poly-)glycerol partial ester, a (poly-)glycerol is obtained which has a homologue distribution with:

glycerol: 0.01% by weight to 20% by weight,
diglycerols: 0.01% by weight to 60% by weight,
triglycerols: 0.01% by weight to 60% by weight,
tetraglycerols: 0.01% by weight to 30% by weight,
pentaglycerols: 0.01% by weight to 20% by weight and
oligoglycerols: ad 100% by weight.

**2.** (Poly-)glycerol partial ester according to Claim 1, **characterized in that** the polyfunctional carboxylic acid is citric acid and comprises citric acid ester (poly-)glycerol partial ester of the general formula (I):

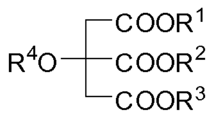 formula (I),

where $R^1$, $R^2$, $R^3$ and $R^4$, independently of one another, are identical or different and are selected from

H or
a radical of the general formula (II)

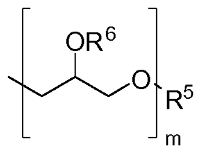 formula (II),

where m is greater than or equal to 1 and $R^5$ and $R^6$, independently of one another, are identical or different and are selected from
H or
an acyl radical having 10 to 24 carbon atoms, where
the acyl radical is determined by the acyl radical of the carboxylic acid esterified with the (poly-)glycerol

and on average (number-average) at least one of the radicals $R^5$ or $R^6$ = H,
with the proviso that on average (number-average) at least one radical $R^1$, $R^2$ or $R^3$ is not H.

**3.** (Poly-)glycerol partial ester according to Claim 1 or 2, **characterized in that**, following complete hydrolysis of the partial ester, the molar ratio of the resulting carboxylic acids derived from $R^5$ and $R^6$ to (poly-)glycerol is between 2:3 and 4:1.

**4.** (Poly-)glycerol partial ester according to at least one of Claims 1 to 3, **characterized in that** the ratio of saponification value to hydroxyl value is between 1:1.3 and 1:2.6.

**5.** (Poly-)glycerol partial ester according to at least one of Claims 1 to 4, **characterized in that** the acid value is less than 50.

**6.** (Poly-)glycerol partial ester according to at least one of Claims 1 to 5, **characterized in that** it has a melting point greater than 35°C.

**7.** (Poly-)glycerol partial ester according to at least one of Claims 1 to 6, **characterized in that** the acyl radical determining $R^5$ and $R^6$ arises from a mixture of stearic acid and palmitic acid in the weight ratio from 30:70 to 95:5.

**8.** Process for the preparation of a (poly-)glycerol partial ester comprising the process steps

A) provision of a (poly-)glycerol having a homologue distribution with

glycerol: 0.01 to 20% by weight
diglycerols: 0.01 to 60% by weight
triglycerols: 0.01 to 60% by weight
tetraglycerols: 0.01 to 30% by weight
pentaglycerols: 0.01 to 20% by weight
oligoglycerols: ad 100% by weight.

B) esterification of some of the (poly-)glycerol with one or more carboxylic acid having 10 to 24 carbon atoms and
C) further esterification with a polyfunctional carboxylic acid,

where the molar ratio of the polyfunctional carboxylic acid used in process step C) to (poly-) glycerol used in process step A) is 1:2 to 1:200.

**9.** Process according to Claim 8, **characterized in that** the molar ratio of the one or more carboxylic acids to (poly-)glycerol used is 2:3 to 4:1.

**10.** Process according to Claim 8 or 9, **characterized in that** the polyfunctional carboxylic acid is citric acid and the carboxylic acid is a mixture of stearic acid and palmitic acid in the weight ratio from 30:70 to 95:5.

**11.** Use of the (poly-)glycerol partial ester according to at least one of Claims 1 to 7 or of the (poly-)glycerol partial ester obtainable by a process according to at least one of the Claims 8 to 10 as emulsifier.

**12.** Use of the (poly-)glycerol partial ester according to at least one of Claims 1 to 7 or of the (poly-)glycerol partial ester obtainable by a process according to at least one of Claims 8 to 10 for producing cosmetic or pharmaceutical formulations.

**13.** Emulsifier comprising (poly-)glycerol partial ester according to at least one of Claims 1 to 7 or (poly-)glycerol partial ester obtainable by a process according to at least one of Claims 8 to 10.

**14.** Use of the (poly-)glycerol partial ester according to at least one of Claims 1 to 7 or of the (poly-)glycerol partial ester obtainable by a process according to at least one of Claims 8 to 10 or of an emulsifier according to Claim 13 for improving the water resistance of cosmetic or pharmaceutical formulations.

**15.** Cosmetic or pharmaceutical formulation comprising (poly-)glycerol partial ester according to at least one of Claims 1 to 7 or (poly-) glycerol partial ester obtainable by a process according to at least one of Claims 8 to 10.

**16.** Cosmetic or pharmaceutical formulation according to Claim 14, **characterized in that** the (poly-)glycerol partial ester is present in amounts of from 0.01 to 10% by weight.

**17.** Cosmetic or pharmaceutical formulation according to Claim 14 or 15, **characterized in that** organic acids are present as preservative in a range from 0.01 to 1.0% by weight.

**18.** Cosmetic or pharmaceutical formulation according to at least one of Claims 14 to 16, **characterized in that** they have a pH of from 3.5 to 5.5.

## Revendications

**1.** Ester partiel de (poly-)glycérol comportant en moyenne (moyenne en nombre) de 0,75 à 2,25 radicaux acides d'un ou de plusieurs acides carboxyliques ayant de 10 à 24 atomes de carbone et comportant en moyenne (moyenne en nombre) de 0,005 à 0,5 radical d'un acide carboxylique polyfonctionnel, en particulier d'acides gras dimères, d'acide oxalique, d'acide fumarique, d'acide malonique, d'acide succinique, d'acide glutarique, d'acide adipique, d'acide pimélique, d'acide subérique, d'acide azélaïque, d'acide sébacique, d'acide dodécanedioïque, d'acide malique, d'acide d-tartrique, d'acide tartronique, d'acide maléique, d'acide citrique, d'acide phtalique, d'acide isophtalique et d'acide téréphtalique, contenant
des esters partiels de (poly-)glycérol d'esters d'acides carboxyliques polyfonctionnels,

étant entendu qu'après hydrolyse totale de l'ester partiel de (poly-)glycérol on obtient un (poly-)glycérol qui présente une distribution d'homologues comportant :

glycérol : 0,01 % en poids à 20 % en poids,
diglycérols : 0,01 % en poids à 60 % en poids,
triglycérols : 0,01 % en poids à 60 % en poids,
tétraglycérols : 0,01 % en poids à 30 % en poids,
pentaglycérols : 0,01 % en poids à 20 % en poids et
oligoglycérols : complément à 100 % en poids

**2.** Ester partiel de (poly-)glycérol selon la revendication 1, **caractérisé en ce que** l'acide carboxylique polyfonctionnel représente l'acide citrique et **en ce qu'**il contient des ester partiels de (poly-)glycérol d'esters d'acide citrique de formule générale (I) :

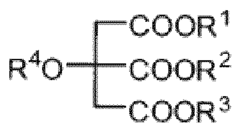
formule (I),

dans laquelle $R^1$, $R^2$, $R^3$ et $R^4$, identiques ou différents, sont choisis indépendamment les uns des autres parmi

H ou
un radical de formule générale (II)

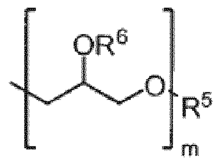
formule (II),

dans laquelle m est supérieur ou égal à 1 et $R^5$ et $R^6$, identiques ou différents, sont choisis indépendamment l'un de l'autre parmi

H ou
un radical acyle ayant de 10 à 24 atomes de carbone,
le radical acyle étant déterminé par le radical acyle de l'acide carboxylique estérifié par le (poly-)glycérol

et en moyenne (moyenne en nombre) au moins un des radicaux $R^5$ et $R^6$ est égal à H,

étant entendu qu'en moyenne (moyenne en nombre) au moins un radical $R^1$, $R^2$ ou $R^3$ est différent de H.

**3.** Ester partiel de (poly-)glycérol selon la revendication 1 ou 2, **caractérisé en ce qu'**après hydrolyse totale de l'ester partiel, le rapport molaire des acides carboxyliques obtenus, dérivés de $R^5$ et $R^6$, au (poly-)glycérol est compris entre 2:3 et 4:1.

**4.** Ester partiel de (poly-)glycérol selon au moins l'une des revendications 1 à 3, **caractérisé en ce que** le rapport de l'indice de saponification à l'indice d'hydroxyle est compris entre 1:1,3 et 1:2,6.

**5.** Ester partiel de (poly-)glycérol selon au moins l'une des revendications 1 à 4, **caractérisé en ce que** l'indice d'acide est inférieur à 50.

**6.** Ester partiel de (poly-)glycérol selon au moins l'une des revendications 1 à 5, **caractérisé en ce qu'**il présente un point de fusion supérieur à 35 °C.

**7.** Ester partiel de (poly-)glycérol selon au moins l'une des revendications 1 à 6, **caractérisé en ce que** le radical acyle déterminant $R^5$ et $R^6$ résulte d'un mélange d'acide stéarique et d'acide palmitique en un rapport pondéral de 30:70

à 95:5.

**8.** Procédé pour la préparation d'un ester partiel de (poly-)glycérol, comprenant les étapes de processus

A) disposition d'un (poly-)glycérol présentant la distribution d'homologues comportant

glycérol : 0,01 à 20 % en poids
diglycérols : 0,01 à 60 % en poids
triglycérols : 0,01 à 60 % en poids
tétraglycérols : 0,01 à 30 % en poids
pentaglycérols : 0,01 à 20 % en poids
oligoglycérols : complément à 100 % en poids,

B) estérification d'une partie du (poly-)glycérol par un ou plusieurs acides carboxyliques ayant de 10 à 24 atomes de carbone et
C) estérification plus poussée par un acide carboxylique polyfonctionnel,

le rapport molaire de l'acide carboxylique polyfonctionnel utilisé dans l'étape C) du procédé au (poly-)glycérol utilisé dans l'étape A) du procédé valant de 1:2 à 1:200.

**9.** Procédé selon la revendication 8, **caractérisé en ce que** le rapport molaire utilisé d'un ou de plusieurs acides carboxyliques au (poly-)glycérol vaut de 2:3 à 4:1.

**10.** Procédé selon la revendication 8 ou 9, **caractérisé en ce que** l'acide carboxylique polyfonctionnel est l'acide citrique et l'acide carboxylique est un mélange d'acide stéarique et d'acide palmitique en un rapport pondéral de 30:70 à 95:5.

**11.** Utilisation de l'ester partiel de (poly-)glycérol selon au moins l'une des revendications 1 à 7 ou de l'ester partiel de (poly-)glycérol pouvant être obtenu conformément à un procédé selon au moins l'une des revendications 8 à 10, en tant qu'émulsifiant.

**12.** Utilisation de l'ester partiel de (poly-)glycérol selon au moins l'une des revendications 1 à 7 ou de l'ester partiel de (poly-)glycérol pouvant être obtenu conformément à un procédé selon au moins l'une des revendications 8 à 10, pour la fabrication de compositions cosmétiques ou pharmaceutiques.

**13.** Émulsifiant, contenant un ester partiel de (poly-)glycérol selon au moins l'une des revendications 1 à 7 ou un ester partiel de (poly-)glycérol pouvant être obtenu conformément à un procédé selon au moins l'une des revendications 8 à 10,

**14.** Utilisation de l'ester partiel de (poly-)glycérol selon au moins l'une des revendications 1 à 7 ou de l'ester partiel de (poly-)glycérol pouvant être obtenu conformément à un procédé selon au moins l'une des revendications 8 à 10, ou d'un émulsifiant selon la revendication 13, pour l'amélioration de la résistance à l'eau de compositions cosmétiques ou pharmaceutiques.

**15.** Composition cosmétique ou pharmaceutique, contenant un ester partiel de (poly-)glycérol selon au moins l'une des revendications 1 à 7 ou un ester partiel de (poly-)glycérol pouvant être obtenu conformément à un procédé selon au moins l'une des revendications 8 à 10.

**16.** Composition cosmétique ou pharmaceutique selon la revendication 14, **caractérisée en ce que** l'ester partiel de (poly-)glycérol est contenu en des quantités de 0,01 à 10 % en poids.

**17.** Composition cosmétique ou pharmaceutique selon la revendication 14 ou 15, **caractérisée en ce qu'**en tant que conservateur des acides organiques sont contenus dans une plage de 0,01 à 1,0 % en poids.

**18.** Composition cosmétique ou pharmaceutique selon au moins l'une des revendications 14 à 16, **caractérisée en ce qu'**elle présente un pH de 3,5 à 5,5.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0835862 B
- WO 2006034992 A
- WO 2008092676 A
- WO 2004112731 A
- DE 4409569
- EP 451461 A
- WO 0172683 A
- EP 1683781 A
- DE 102008001788


### In der Beschreibung aufgeführte Nicht-Patentliteratur


- **CASSEL et al.** Original synthesis of linear, branched and cyclic oligoglycerol standards. *Eur. J. Org. Chem.,* 2001, 875-896
- **HANDBÜCHER, Z. ; B. K. SCHRADER.** Grundlagen und Rezepturen der Kosemtika. 329-341
- *Int. J. Cosm. Sci.,* 2007, vol. 29, 451-460